# EUROPEAN PATENT APPLICATION

(11) **EP 4 364 668 A1**
(43) Date of publication of application: **08.05.2024**
(21) Application number: 22205215.1
(22) Date of filing: 03.11.2022
(51) Int. Cl.: A61B 6/04, A61B 6/00

(54) **A DEVICE, COMPUTER PROGRAM PRODUCT AND METHOD FOR ASSISTING IN POSITIONING AT LEAST ONE BODY PART OF A PATENT FOR AN X-RAY ACQUISITION**

(71) Applicant: Koninklijke Philips N.V., 5656 AG Eindhoven (NL)
(72) Inventor: VON BERG, Jens, Eindhoven (NL); YOUNG, Stewart Matthew, Eindhoven (NL); BRUECK, Heiner Matthias, 5656AG Eindhoven (NL); KROENKE-HILLE, Sven, 5656AG Eindhoven (NL); GUENZEL, Dominik, 5656AG Eindhoven (NL); BYSTROV, Daniel, 5656AG Eindhoven (NL); GOOßEN, Andre, 5656AG Eindhoven (NL); HARDER, Tim Philipp, 5656AG Eindhoven (NL)
(74) Representative: Philips Intellectual Property & Standards

(57) **Abstract**

The invention concerns a device, a method, and an X-ray-system comprising such a device, for assisting in positioning at least one body part of a patient for an X-ray acquisition by an X-ray system, comprising a display for displaying the body part to a user, a first camera on a first side of the display, arranged and configured to monitor the body part, wherein the body part is monitored from a viewing perspective of the device, and a processing unit. The processing unit is configured to estimate an actual bone position of the body part based on one or more images captured by the first camera, determine a target bone position, calculate a difference between the estimated actual bone position and the target bone position. The device is configured to display by the display at least one of the target bone position, the estimated actual bone position, and the calculated difference between the estimated actual bone position and the target bone position overlaid over at least one image of the body part. The device is configured to adapt the displaying of the at least one of the target bone position, the estimated actual bone position, and the difference between the estimated actual bone position and the target bone position upon a change of the viewing perspective of the device.

## Description

### FIELD OF THE INVENTION

The invention relates to the field of X-ray acquisition, in particular to the field of positioning a patient for an X-ray acquisition. More specifically, the invention relates to a device for assisting in positioning of a body part of a patient to be imaged by an X-ray system, an X-ray system comprising such a device, a computer program product and a method for assisting in positioning at least one body part of a patient for an X-ray acquisition.

### BACKGROUND OF THE INVENTION

In practice, the exact patient positioning, i.e. the exact position of a body part of a patient, for musculoskeletal X-ray acquisitions is critical for the diagnostic quality of the X-ray images. Recently methods have been developed to image a patient being positioned for an X-ray acquisition, to estimate the actual position of the bones from that image, and to align that actual position with the expected ideal pose to be taken for the X-ray acquisition. During the acquisition, the technician has to manage and locate the examination table, the X-ray system, for instance the X-ray tube head, and the patient body part while taking care for the patient.

In general, a camera for viewing the patient is attached to a tube head of an X-ray tube. This camera is a part of the X-ray system. Displaying of the observed scene is done on the display at the tube head, at a console display or on a separate display at a wall of the examination room. The user, i.e. the technician, the medical staff, has to manage, monitor and locate the examination table, the external display at the wall, the tube head, and the patient body part while taking care of the patient and while arranging the patient for the X-ray acquisition.

If a position of a patient or a position of a body part of a patient is not adequate, not correct, a retake of an image is required, wherein for this retake the technician has to consider the position of the patient, the position of the X-ray system, and at least the display at the wall (or the tube head) of the examination room.

Therefore, there exist a need for showing to the user, the technician, the body part to be positioned a in a more natural manner and less complex and guiding the user in an easier and intuitive way for positioning the body part of the patient.

There exit the question how the information for positioning the patient is best shown to the operator, the medical personal, the radio-technician so that this person is not constrained in the workflow.

### SUMMARY OF THE INVENTION

There exist a need for an easier and more natural positioning support of at least a body part of a patient for an X-ray acquisition for the user. In particular, there exists a need in an easier guidance, assistance, for a user for positioning a body part of a patient such that the examination personal is able to perform less managing steps for the positioning of a patient at an examination table, and an easier displaying of a positioning of a body part to be positioned.

The object of the present invention is solved with the subject matter of the independent claims, wherein further embodiments are incorporated in the dependent claims.

An object of the invention is improving a positioning of at least a body part of a patient for an X-ray acquisition, in particular improving an assistance of positioning of at least a body part of a patient for an X-ray acquisition. In particular, improving guidance for a user for an improved displaying of a positioning of a body part and positioning a body part of a patient.

According to a first aspect of the invention, a device is described for assisting in positioning at least one body part of a patient for an X-ray acquisition by an X-ray system, comprising a display for displaying the body part to be positioned to a user operating the device, a first camera on a first side of the display, arranged and configured to monitor the body part of the patient to be positioned, wherein the body part is monitored from a viewing perspective of the device, and a processing unit. The processing unit is configured to estimate an actual bone position of the body part based on one or more images captured by the first camera, determine a target bone position, calculate a difference between the estimated actual bone position and the target bone position. The device is configured to display by the display at least one of the target bone position, the estimated actual bone position, and the calculated difference between the estimated actual bone position and the target bone position overlaid over at least one image of the body part. The device is further configured to adapt the displaying of the at least one of the target bone position, the estimated actual bone position, and the difference between the estimated actual bone position and the target bone position upon a change of the viewing perspective of the device.

In the context of the present invention, the term "X-ray acquisition" shall be understood to describe gaining information about a patient, at least about a body part of the patient during X-ray imaging. In other words, receive X-ray data, preferably, X-ray image data of a patient. For this X-ray acquisition, a patient, or rather the body part of the patient has to be positioned in the right manner, such that the information received by the X-ray acquisition are sufficient, good enough for being analyzed by a medical person.

In the context of the present invention, the term "assisting in positioning" shall be understood to describe that the device may be used for helping the user of the device, which may be a medical person, preparing the patient for the X-ray acquisition. The assisting may be guiding the user to position the body part of the patient in the right and correct manner, such that an accurate X-ray image could be made of the body part after the positioning of the patient. Accordingly, the device may be used as an instruction for guiding the user for positioning the patient/body part of the patient, or the device may show an instruction to the user for the correct positioning.

In the context of the present invention, the term "viewing perspective" shall be understood to describe a perspective from a first point of view to a second point of view, wherein the viewing direction extends from the first to the second point of view. In the described embodiments, the viewing perspective extends between the body part and the device and/or the user of the device, wherein the user of the device may be indirectly the first viewing point because the user holds the device and points with the device into the direction of the body part to be positioned. In the described embodiments herein, the second point of view may be the body part, which should be positioned, hence the viewing perspective is directed towards the body part. Accordingly, the viewing perspective of the device is a perspective of the device, which is indirectly the viewing perspective of the user, who is arranging the device, into the direction of the body part from any spatial angle, which might be suitable for a best viewing for a respective, desired pose, i.e. position of the body part.

In the context of the present invention, the term "target bone position" shall be understood to describe a position of bones of the body part which are suitable for an X-ray acquisition, wherein when the target bone position is reached by the patient, a resetting, a rearrangement of the body part is not necessary. Hence, the arrangement procedure during the X-ray inspection can be simplified. In other words, the target bone position may be understood as a desired pose of the body part for a respective diagnostic image. The use of optimal target bone positions results in optimal X-ray images, wherein there exists different target bone positions depending on the body part to be X-rayed and depending on particular diagnostic request.

In the context of the present invention, the term "actual bone position" shall be understood to describe a position of the body part, which has been taken in the present moment. In other words, the actual posture taken by the patient, the actual position of the body part, which actually is taken by the patient. On the one hand, depending on the arrangement of the patient the actual bone position may be a position of the body part which is not correct. Therefore, the body part has to be rearranged in its position. On the other hand, the actual bone position may correspond the desired target bone position, when the body part already has been re-positioned.

In this embodiment, the device is proposed to monitor the position of the patient, in particular the position of the body part of the patient and estimate bone positions in the reference system of the device in real time. Ideal target bone positions for the acquisition may be defined and transferred into the reference system of the device. The difference between both the adjustment to be made is calculated and augments the image displayed on the device. The displaying may comprise a displaying of an image and/or of a video, i.e. a sequence of images. The device, which may be held in front of the patient body part, which should be positioned, gives a very natural hint how to adjust a position of this body part from any arbitrary perspective and easily suits into the positioning workflow. In this embodiment, the device may also be able to display simultaneously by the display the target bone position, the estimated actual bone position and the calculated difference between the estimated actual bone position and the target bone position in an overly manner. Accordingly, the display may not only display at least one of the different positions (actual bone position, target bone position, and difference between these two position) but also two of them or all of them and display them in, or just one of them, in an overlaid manner on the display to the user. In particular, the processing unit may be configured to reconstruct the scene of a viewing perspective of the device (or the user) by aid of the first camera. The displayed position is changeable depending on the viewing perspective of the device. This means, if the user changes the perspective of the device directed to the body part, hence if the user changes his perspective on viewing to the body part, the device is configured to adapt the change in the perspective directly and in real time, such that the displayed information on the display are adapted upon the change of the perspective accordingly. After comparing the target bone position with the actual bone position the difference, i.e. a deviation, can be calculated. The simultaneously displaying of at least one pose and the image of the body part of the patient can be done in live modus, which means in real time. Therefore, the live position of the patient can be monitored and analyzed and can be repositioned directly. A suitable combination of the target bone position, the actual bone position and the changes between both are to be displayed on the display of the device from the perspective of the device and is aligned with the live image(s).Accordingly, with the device it is possible that the body part which should be positioned can be viewed in a more natural way to the user, such that there is no need to observe the examination table, the tube head display or a wall display at the same time. With this, the monitoring of the positioning of the body part is made easier. Further, the device allows to guide, assist, the user for positioning the body part in a more intuitive manner, wherein also the steps of analyzing the examination table and the body part placed thereon and the tube head (display) and a wall display can be omitted. The body part of the patient is displayed to the user of the device in a natural perspective, which eases the steps of positioning of the body part. The user is able to approach the body part from any side and can fine tune its position from there. In order to do so, augmented reality overlays may displayed, on top of the live image of the body part by the display. As they are aligned with the image of the display and also appear from the perspective of the display (and hence from the users perspective) they are very intuitive to follow.

The use of the device may be convenient for the user. In general, the device may be used any time wherever the user is in the acquisition room. At least one hand is needed for the positioning of the body part and the other hand may held the device, such that a closed loop feedback in fine positioning is guaranteed until the desired position is achieved. The feedback may be given directly during the positioning visual or haptic. With the device as described, a user can easily adapt himself to work from a different desired position than the tube position that may be even more conclusive for positioning and at the same time more convenient for user and patient.

If the target bone position and the estimated actual bone position matches, hence these both position are the similar, hence there is no, or almost no difference between these two positions determined, the desired position (target bone position) is reached. After reaching the target bone position with the assisted positioning of the body part, the X-ray acquisition may be carried out in a sufficient manner.

It should be noted that any feature, function and/or element described hereinabove and/or in the following with reference to the device equally applies to the system and/or the method, and vice versa. Accordingly, any feature, function, step and/or element described in the following with reference to one aspect of the present disclosure equally applies to any other aspect of the present disclosure.

According to an exemplary embodiment of the invention, the viewing perspective of the device may comprises a spatial perspective between the viewing direction of the first camera into the direction of the body part. Therefore, when the perspective of the device is changed, for example by the operation of the user, the spatial perspective, which spans an optical axis between the first camera and the body part, changes and the body part is viewed from another point of view. When this happens, the device is able to again estimate, calculate, monitor etc. the respective position of the bones of the body part in real time and display this to the user for a correct positioning of the body part.

According to an exemplary embodiment of the invention, the device may further comprise a second camera on a second side of the display, arranged and configured to monitor a viewing perspective of the user, wherein the viewing perspective of the user comprises a spatial perspective between the viewing direction of the user and the device into the direction of the body part. The second camera and the first camera may be arranged on opposite sides of the display, such that the first camera comprises a view into the direction of the body part and the second camera comprises a view into the direction of the user. Accordingly, the first camera captures at least one image of the body part and the second camera captures at least one image of the user. The first camera may be configured to monitor the body part of the patient from the user's perspective, wherein the first camera is arranged during the use at an imaginary line forming the viewing perspective from the user to the body part.

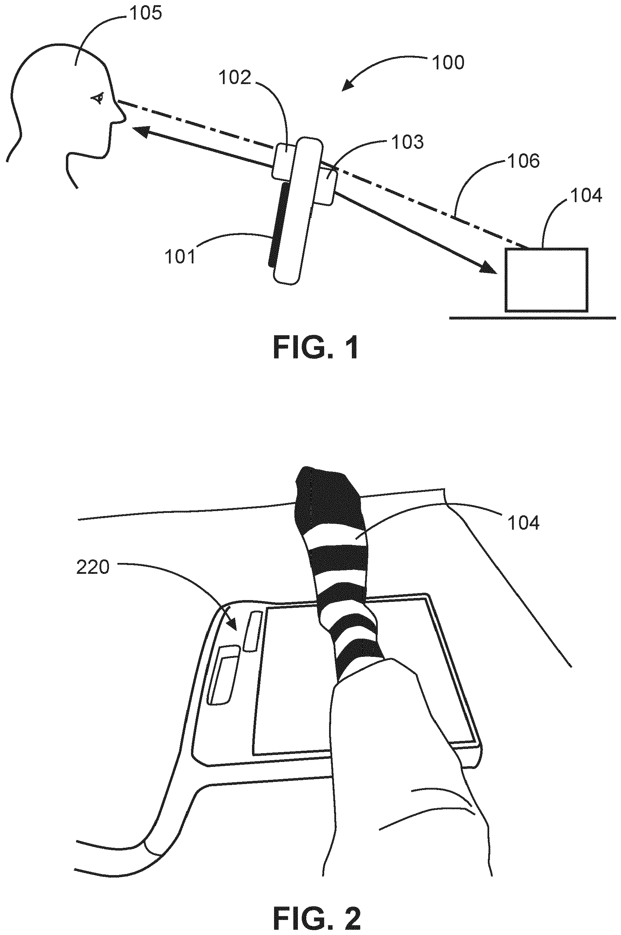

Both cameras may allow to zoom in and out, for instance when displacing the device along the optical axis of the viewing perspective between the body part and the device. The second camera may be aligned with the display and the user, such that the display gives a very natural impression as continuation part of the operators view. A more detailed description may be described with Fig. 1.

According to an exemplary embodiment of the invention, the first camera may generate one or more images, wherein the processing unit is configured to estimate the actual bone position of the body part based on the one or more images of the first camera. In particular, the images generated by the camera may be used to show to the user via the display at least one image, or an image sequence, or a video, or the user may choose what should be displayed. Preferably, the generated images are a sequence of images, a video, which is displayed via the display to the user and which is a live video of the body part, which should be positioned for the X-ray acquisition. The processing unit may be configured to estimate the actual bone position based on or more RGB images, and/or RGBd images (images comprising depth information). The RGBd image(s) from the first camera may be used to estimate the pose, position, from for example a joint and its part. Further, the images may be any images, which could be used for extracting depth information for generating a bone position. In particular, time-of-flight cameras may be used for the first and/or the second camera of the device, because they may be provide proper depth information in the image. For instance, the actual bone position may be estimated from the 3D surface of the body part, wherein the surface of the body part is analyzed from the images taken by the first camera of the device.

According to an exemplary embodiment of the invention, the second camera may generate one or more images, wherein the processing unit may be configured to estimate the viewing direction, viewing origin, and/or eyes of the user based on the one or more images of the second camera. The estimation of the first and the second camera may carried out with reference to the device coordinate system, such that the device itself may serve as a common reference. Then the estimated actual bone position may be overlaid to the live image of the body part.

According to an exemplary embodiment of the invention, for determining the target bone position the processing unit may be further configured to determine a position of an X-ray source and a position of an X-ray detector of an X-ray system used for the X-ray acquisition, wherein the position is determined with reference to an X-ray coordinate system. The determining of the target bone position may be carried out by the processing unit itself on the other hand the determination may be cloud based in such a manner, that the processing unit is able to access data relating to known target bone positions, which are typically used during X-ray acquisitions depending on the respective body part (the knee, limp, joint, feet, hand and so on). Accordingly, the processing unit may use external data for determining a target bone position. In the context of the present invention, the reference with respect to the X-ray coordinate system may be understood to describe, that all parts of the X-ray system are arranged with respect to each other, wherein these parts are spatially arranged to each other in a respective coordinate system, which is the X-ray coordinate system. Hence, the parts of the X-ray system may be arranged and located in relation to each other in the X-ray system. The determination of the X-ray source position and the X-ray detector position may be determined in the X-ray reference system either through prior calibration via external markers and/or by using an in-built gyroscopic tracking of the device position. The reconstructed scene may also be analyzed and judged in this X-ray reference system for which reference positions, i.e. target bone positions are available based on guidelines and/or training data. When determining the target bone position with reference in the X-ray coordinate system, it may be guaranteed, that the target bone position is a correct position of the body part, which is an optimal position for the X-ray acquisition.

According to an exemplary embodiment of the invention, the processing unit may be configured to determine at least one of a position of the display, an orientation of the display, with reference to the X-ray coordinate system such that a position of the display with respect to the X-ray system can be determined. In other words, the position and orientation of the device, in particular of the display, are localized with respect to the X-ray coordinate system, in particular with respect to the X-ray source and/or the X-ray detector, or vice versa. The localization of the device, or parts therefrom, in the X-ray coordinate system allows the device to define a reference to the X-ray system and to link the respective perspective of the device to the perspective of the X-ray system (the perspective of the X-ray system to the body part) and whether the position of the body part is sufficient for the X-ray system for performing a good X-ray acquisition.

According to an exemplary embodiment of the invention, the processing unit may be configured for transforming the estimated actual bone position of the body part into the X-ray coordinate system and is configured to compare the estimated actual bone position with the target bone position in the X-ray coordinate system. Further, the processing unit may be configured for transforming the estimated actual bone position in the X-ray coordinate system and the target bone position in the X-ray coordinate system into the device coordinate system, such that the positions are displayable via the display from the viewing perspective of the device. In other words, the processing unit may be configured to transform one or more bone position in the X-ray coordinate system and on the other hand may be configured to transform one or more bone positions into the device coordinate system or vice versa, wherein both transformations may be carried out independent from each other simultaneously or separately one after another. The estimated actual bone position(s) is transformed into X-ray coordinates and their position may be compared with the target bone position, wherein the target bone position may already be transformed into the X-ray coordinates or the device may also transform the target bone position into X-ray coordinates. After the comparison, the device may indicate whether the estimated actual bone position corresponds to the target bone position or not. If the actual bone position is not the same as the target bone position the difference between both positions may be calculated, wherein the difference between these positions is also determined with reference to the X-ray coordinate system. Afterwards all three bone positions, i.e. actual bone position, target bone position and differences between both, are transformed back into device coordinates to be displayed and aligned in an overlaid manner on the live image of the body part on the display. The transformation of bone positions into the X-ray coordinate system allows the comparison with target bone position, which are already X-ray coordinate format. The transformation into the device-coordinate system allows to produce a reference to the perspective of the device, accordingly to the perspective of the user, such that an adaption on a changed device perspective (changed user perspective) can be made possible.

According to an exemplary embodiment of the invention, the processing unit may be further configured to generate an articulated model of the imaged body part anatomy or geometric primitives indicating joint pose parameters. The device may be configured to indicate the estimated actual bone position with at least one of an articulated model of the imaged body part anatomy or geometric primitives indicating joint pose parameters via the display. For instance, the displaying of the actual bone position may be done by using the articulated model of the imaged anatomy, which shows a bone model simultaneously overlaid over the image of the body part. In addition or separately, some geometric primitives representing joint properties may be determined and displayed in an overlaid manner over the image of the body part, in particular over the live image of the body part. These geometric primitives may be one or more axes, representing bones or limbs.

According to an exemplary embodiment of the invention, the device may be configured for providing a feedback to the user with respect to the target bone position and the difference between the actual bone position and the target bone position for positioning the body part of the patient. The feedback may be a haptic feedback, for example by vibration whether the target bone position is reached, i.e. whether the actual bone position matches the target bone position. The feedback may be provided to the user in real time. The haptic feedback may depend upon the agreement between the scene shown on the live image and the desired configuration, and is intended to guide the user, the operator, towards its desired configuration in order to support them to obtain optimal positioning of the body part with respect to an anatomical model. The feedback may also be a visual feedback. For example, the feedback may be the indicated difference between the actual bone position and the target bone position, which may be indicated by an arrow showing the direction into which the user has to adjust the position or ankle of the body part for an optimal position of the body part. This difference may be adapted in real time, when the perspective of the device and/or of the user changes and when the body part is repositioned.

According to an exemplary embodiment of the invention, the second camera may be arranged and configured to track eyes of the user operating the device for determining the viewing perspective of the user. In particular, the second camera may track the eyes of the user in order to render the image of the first camera in a way representing a natural continuation of the user's visual field. In this way, bringing the device closer to the eyes is a very natural way of zooming out and vice versa.

According to an exemplary embodiment of the invention, the first camera may be positioned opposite to the display center, such that a parallax between the display and the first camera is reduced. In other words, the first camera and the display center are arranged on the same optical axis. In addition, the first camera, and the second camera and the display center may be positioned on the same optical axis, such that a parallax between these three points is reduced. In particular, the first camera and the second camera may be arranged on sides of the display opposite to each other. The position of both cameras to each other may be stiff or at least well determined in order to achieve a correct augmented reality impression, which displayed to the user via the display in an overlaid manner.

According to an exemplary embodiment of the invention, the device may further comprise a wristband, wherein the display may be arranged at the wristband, and wherein the first camera may be arranged at the wristband. When arranging the device at a wristband, this allows for the user that both hands may be free for the X-ray acquisition, and during the positioning of the body part only one hand may be used for positioning.

According to a second aspect of the invention, a computer program product is described, for assisting in positioning at least one body part of a patient for an X-ray acquisition. The computer program element, when being executed by a processor of a device for positioning at least a body part of a patient presented herein, is adapted to cause the device to monitor the body part via a first camera of the device, wherein the body part is monitored from a viewing perspective of the device, to estimate an actual bone position of the body part based on one or more image captured by the first camera, to determine a target bone position, to calculate a difference between the estimated actual bone position and the target bone position, to display by the display at least one of the target bone position, the estimated actual bone position, and the calculated difference between the estimated actual bone position and the target bone position overlaid over at least one image of the body part, to adapt the displaying of the at least one of the target bone position, the estimated actual bone position, and the difference between the estimated actual bone position and the target bone position upon a change of the viewing perspective of the device. The processor of this embodiment may be similar or equal to the processing unit as described with the other embodiments.

In this embodiment the computer program product may estimate the actual bone position based on RGB, and/or RGBd images as described in the embodiments hereinabove. The computer program element, when being executed by the processor of the device for positioning at least the body part of the patient presented herein, may be further adapted to determine a position and an orientation of a display of the device with reference to an X-ray coordinate system, transform the estimated actual bone position into X-ray coordinates, compare the estimated actual bone position with a target bone position, and transfer at least one of the estimated actual bone position, the target bone position, and a difference between the estimated actual bone position and the target bone position into a device coordinate system.

According to a third aspect of the invention an X-ray system is described, comprising an X-ray source, an X-ray detector for generating an X-ray image of at least a body part of a patient, a device for positioning the body part of the patient for an X-ray acquisition according to any one of the embodiments as described hereinabove. The device may be configured for determining a position of the X-ray detector and a position of the X-ray source. In this embodiment the X-ray system may be equipped with such a device according to one or more embodiments as described herein above, such that the device is capable to assist in positioning the patient for the X-ray acquisition.

According to a fourth aspect of the invention a method is described for assisting in positioning at least one body part of a patient for an X-ray acquisition, the method comprising the following steps. Operating a device for positioning the at least one body part of the patient for the X-ray acquisition. Monitoring the body part to be positioned via a first camera, wherein the body part is monitored from a viewing perspective of the device. Estimate an actual bone position of the body part based on one or more image captured by the first camera. Determine a target bone position. Calculate a difference between the estimated actual bone position and the target bone position. Display by the display at least one of the target bone position, the estimated actual bone position, and the calculated difference between the estimated actual bone position and the target bone position overlaid over at least one image of the body part. Adapt the displaying of the at least one of the target bone position, the estimated actual bone position, and the difference between the estimated actual bone position and the target bone position upon a change of the viewing perspective of the device. In this listing of the steps the order of the steps is not set, hence the order of the steps may be carried out in another order than mentioned herein above. Further, some or all of the steps may be carried out simultaneously or independent from each other. The same applies to the other and/or further method steps mentioned in one or more embodiments of the invention.

According to an exemplary embodiment of the invention, the method may further comprise at least one of the following steps. Determining a position of an X-ray detector and a position of an X-ray source of an X-ray system used for the X-ray acquisition, wherein each of the positions is determined with reference to an X-ray coordinate system. Transforming the estimated actual bone position of the body part into the X-ray coordinate system, Comparing the estimated actual bone position with the target position in the X-ray coordinate system. Transforming the estimated actual bone position in the X-ray coordinate system and the target bone position in the X-ray coordinate system into a device coordinate system, such that the position are displayable via the display from the viewing perspective of the device.

It has to be noted that embodiments of the invention have been described with reference to different subject matters. In particular, some embodiments have been described with reference to apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the apparatus type claims and features of the method type claims is considered as to be disclosed with this application.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 is an illustration of a device according to an embodiment of the invention.
Fig. 2 is an illustration of a perspective of a user according to an embodiment of the invention.
Fig. 3 is an illustration of the device according to an embodiment of the invention.
Fig. 4 is an illustration of a displayed bone position according to an embodiment of the invention.
Fig. 5 is an illustration of a displayed further bone position according to an embodiment of the invention.
Fig. 6 is an illustration of a displayed further bone position according to an embodiment of the invention.
Fig. 7 is an illustration of a target bone positions according to an embodiment of the invention.
Fig. 8 is an illustration of a method according to an embodiment of the invention.

### DETAILED DESCRIPTION OF EMBODIMENTS

The illustrations in the drawings are schematic. It is noted that in different figures similar or identical elements are provided with the same reference signs.

Fig. 1 illustrates a device 100 according to an embodiment of the invention. The device 100 for assisting in positioning at least one body part 104 of a patient for an X-ray acquisition by an X-ray system comprises a display 101 for displaying the body part 104 to a user 105 operating the device 100. The device 100 further comprises a first camera 103 on a first side of the display 101, in particular on a first side of the device 100. The first camera 103 is arranged and configured to monitor the body part 104 of the patient to be positioned, wherein the body part 104 is monitored from a viewing perspective of the device 100 (indicated in the figure by an arrow form the device 100 directed to the body part 104). As can be seen in Fig. 1, the perspective of the device 100 may be indirectly the perspective of the user 106, which is indicated in Fig. 1 with the dotted line 106. The device 100 further comprises a processing unit (not illustrated), which is configured to estimate an actual bone position of the body part 104 based on one or more images captured by the first camera 103, determine a target bone position, calculate a difference between the estimated actual bone position and the target bone position. The device 100 is configured to display by the display 101 at least one of the target bone position, the estimated actual bone position, and the calculated difference between the estimated actual bone position and the target bone position overlaid over at least one image of the body part 104. Further, the device 100 is configured to adapt the displaying of the at least one of the target bone position, the estimated actual bone position, and the difference between the estimated actual bone position and the target bone position upon a change of the viewing perspective of the device 106.

Additionally, the device 100, may comprise a second camera 102, on a second side of the display 101, arranged and configured to monitor a viewing perspective of the user 105, at least on origin of the viewing perspective of the user 105, which is indicated by the arrow from the second camera 102 to the user 105. The second camera 102 may be configured to track the eyes of the user 105. The viewing perspective of the user 106 comprises a spatial perspective between the viewing direction of the user 105 and the device 100 into the direction of the body part 104.

Fig. 2 illustrates the positioning of a body part 104 in an X-ray system 220 from a perspective of the device 106 and/or from a perspective of the user 105 according to an embodiment of the invention. In particular, in this figure the feet of a patient is illustrated positioned on an examination table 220 of the X-ray system. The perspective in the figure may be seen as the perspective, which is suitable for a naturally positioning from the users side, wherein this perspective may also be the perspective of the X-ray system or not.

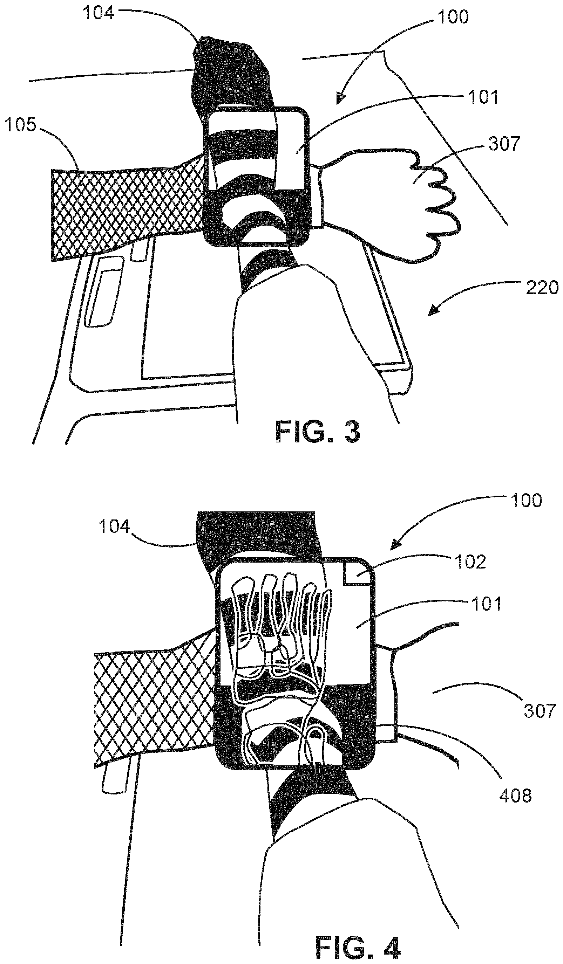

Fig. 3 is an illustration of the device 100 according to an embodiment of the invention, wherein a part of the user 105, its arm and hand, is illustrated. The device is attached to the users 105 hand 307 or wrist, for example by a wrist band (not shown). The display 101 of the device 100 displays a live image of the body part 104, which should be positioned, in this figure the feet ankle of the patient. As can be seen, the patient is positioned on the examination table of the X-ray system 220 and the device 100 displays a live image of the body part 104.

Fig. 4 is an illustration of a displayed bone position 408 according to an embodiment of the invention. The display 101 shows the estimated actual bone position 408 to the user 105 overlaid over the actual image of the body part 104. In particular, the device 100 displays via the display 101 the estimated actual bone position using an illustration of the bones, an articulated model of the imaged anatomy. The image of the body part is captured by the first camera 103, which cannot be seen in this figure. The second camera 102 is attached on the side of the display 101, which faces the user 105, such that this camera is able for tracking perspective of the user 105, and/or the eyes of the user 105.

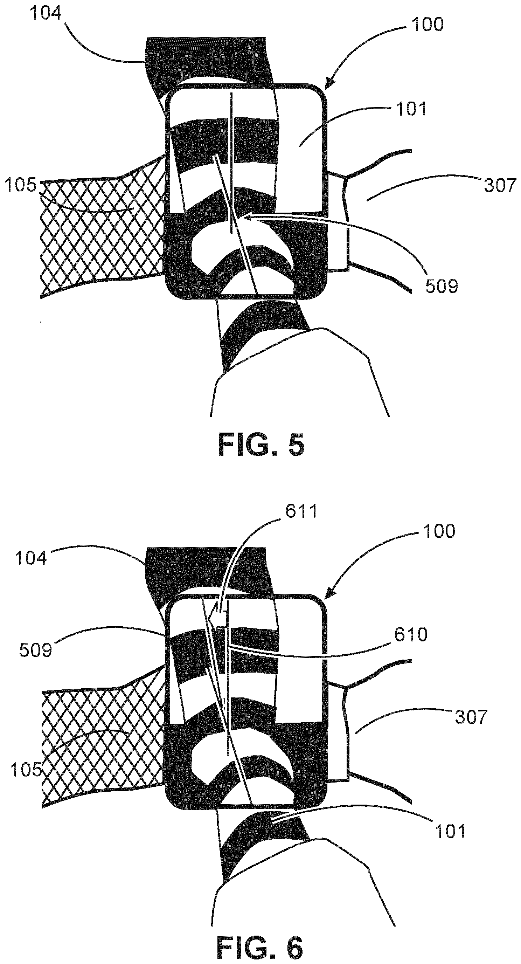

Fig. 5 is an illustration of a displayed further bone position 509 according to an embodiment of the invention. In this figure, the estimated actual bone position is indicated on the display 101 of the device 100 using geometric primitives indicated by joint position parameters. These geometric primitives are crossing lines, indicating an angle of the joint of the body part 104. These geometric primitives 509 are displayed simultaneously with the live image, or video of the body part 104 via the display 101. When the user 105 moves or when the user 105 moves the device 100, the perspective of the view changes and accordingly the illustrated bone position 509 will change, such that a positioning of the body part 104 is possible from any point of view in real time.4

Fig. 6 is an illustration of a further bone position 610, 611 displayed on the display 101 of the device 100 according to an embodiment of the invention. In this figure, three bone positions are displayed on the display 101 to the user 105. The target bone position 610 is indicated by the geometric line 610 and the estimated actual bone position is indicated by the other geometric line on the left side, to which the arrow 611 is pointing. Further, the difference between the actual and the target bone position is indicated by the arrow 611. The user 105 may be able by the simultaneously displaying of all of this bone positions to adapt the position of the body part 104 when checking the estimated actual bone position 611, the target bone position 610 and the difference between these both live during the positioning of the patient on the display 101. When there is no difference between the target bone position 610 and the estimated actual bone position 611 there is no difference (no arrow) displayed, and the lines of the target bone position and the estimated actual bone position would overlap each other, or only one line may be displayed.

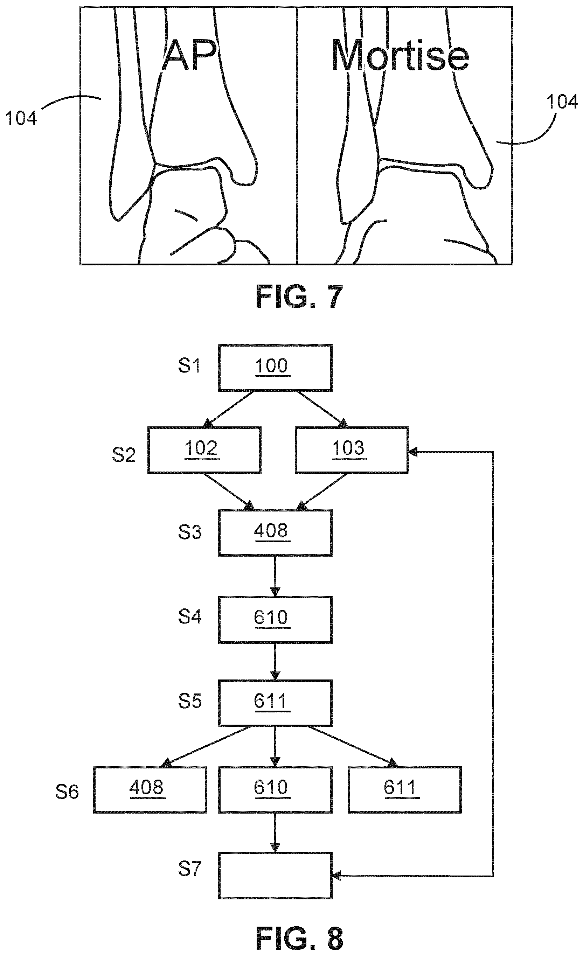

Fig. 7 shows an example of bone positions, in particular of target bone positions. For the example of an angle joint acquisition as illustrated in the other Figures, there exist standard ankle anterior-posterior (AP) or ankle mortise positions indicated with known ranges of position parameters which result in slightly different optimal views in an X-ray image. These target position can be used for comparison with the estimated actual bone position of the body part 104.

Fig. 8 is an illustration of method steps according to an embodiment of the invention. The method described in this figure may be carried out by the device 100 and also by the computer program product as described in the other embodiments of the invention. The method is a method for assisting in positioning at least one body part of a patient for an X-ray acquisition. The method comprises the following steps S1 to S7. In Fig.8 the respective element or unit, which may be used for the step or which performs the step is associated in the respective rectangle in the figure. The steps may be performed in the order as presented in Fig. 8, nevertheless it may be possible to change the order of the steps or exchange one or more steps with other steps. Further, some or all of the steps may be performed simultaneously. The same applies to the other and/or further method steps mentioned in the other embodiments of this invention. Hence, the invention is not limited to the respective order of the steps as shown in the Fig. 8 and as elucidated herein below. The first step S1 comprises operating the device 100 for positioning the at least one body part 104 of the patient for the X-ray acquisition. The next step S2 comprises monitoring the body part 104 to be positioned via a first camera 103, wherein the body part 104 is monitored from a viewing perspective of the device 100. This step S2 may also comprise the monitoring of the user 105 via a second camera 102. Alternatively, the monitoring of the user 105 may be performed as a separate step of this method. A next step S3 comprises estimating an actual bone position 408 of the body part 105 based on one or more image captured by the first camera 103. Next in step S4 a target bone position 610 is determined. After the steps S3 and S4, in step S5 a difference between the estimated actual bone position and the target bone position is calculated. Step S6 comprises displaying by the display 101 at least one of the target bone position 610, the estimated actual bone position 408, and the calculated difference 611 between the estimated actual bone position 408 and the target bone position 610 overlaid over at least one image of the body part 104. In step S6 the different bone positions (target bone position 610, actual bon position 408) or their difference 611 may also be displayed simultaneously or each of them is displayed separately one after another. Nevertheless, all or at least one or some of them are displayed simultaneously with the real time image and/or video of the body part 104 via the display 101. Step 7 comprises adapting the displaying of the at least one of the target bone position 610, the estimated actual bone position 408, and the difference 611 between the estimated actual bone position 408 and the target bone position 611 upon a change of the viewing perspective of the device 100. When step S7 is performed, it is determined whether the perspective has changed and whether the actual bone position 408 has to be estimated again, such that the method may restart from step S2 again. It may also be possible to start from any other suitable step, for example like step S3 the estimation of the actual bone position 408. According to an exemplary embodiment of the invention, the method may further comprise at least one of the following steps. The following steps may also be integrated in one of the steps S1 to S7 as described herein above. Determining a position of an X-ray detector and a position of an X-ray source of an X-ray system used for the X-ray acquisition, wherein each of the positions is determined with reference to an X-ray coordinate system. Transforming the estimated actual bone position 408 of the body part into the X-ray coordinate system, which may be performed in step S3 or wherein S3 may further comprise the transformation step. Another step may be comparing the transformed estimated actual bone position 408 with the target position 610 in the X-ray coordinate system. This might be a separate step of the method illustrated in Fig. 8 or it may be a part of step S4. A further step may be transforming the estimated actual bone position 408 in the X-ray coordinate system and the target bone position 610 in the X-ray coordinate system into a device coordinate system, such that the position(s) is display able via the display 101 from the viewing perspective of the device 100. This transformation into the display coordinate system may also be part of step S4, or it may be a step performed after step 4 and before step 5.

It should be noted that the term "comprising" does not exclude other elements or steps and "a" or "an" does not exclude a plurality. Also elements described in association with different embodiments may be combined. It should also be noted that reference signs in the claims should not be construed as limiting the scope of the claims.

### LIST OF REFERENCE SIGNS:

- 100: device
- 101: display
- 102: first camera
- 103: second camera
- 104: body part
- 105: user
- 106: viewing perspective
- 220: X-ray system
- 307: Users hand
- 408: actual bone position
- 509: actual bone position
- 610: target bone position
- 611: difference between positions

## Claims

1. A device for assisting in positioning at least one body part of a patient for an X-ray acquisition by an X-ray system, comprising:
a display for displaying the body part to be positioned to a user operating the device,
a first camera on a first side of the display, arranged and configured to monitor the body part of the patient to be positioned, wherein the body part is monitored from a viewing perspective of the device,
a processing unit,
wherein the processing unit is configured to
estimate an actual bone position of the body part based on one or more images captured by the first camera,
determine a target bone position,
calculate a difference between the estimated actual bone position and the target bone position,
wherein the device is configured to display by the display at least one of the target bone position, the estimated actual bone position, and the calculated difference between the estimated actual bone position and the target bone position overlaid over at least one image of the body part,
wherein the device is configured to adapt the displaying of the at least one of the target bone position, the estimated actual bone position, and the difference between the estimated actual bone position and the target bone position upon a change of the viewing perspective of the device.

2. A device according to claim 1,
wherein the viewing perspective of the device comprises a spatial perspective between the viewing direction of the first camera into the direction of the body part.

3. A device according to claim 1 or 2, further comprising
a second camera on a second side of the display, arranged and configured to monitor a viewing perspective of the user,
wherein the viewing perspective of the user comprises a spatial perspective between the viewing direction of the user and the device into the direction of the body part.

4. A device according to any one of the preceding claims,
wherein the first camera generates one or more images,
wherein the processing unit is configured to estimate the actual bone position of the body part based on the one or more images of the first camera.

5. A device according to any one of the preceding claims,
wherein for determining the target bone position the processing unit is further configured to determine a position of an X-ray source and a position of an X-ray detector of an X-ray system used for the X-ray acquisition, wherein the position is determined with reference to an X-ray coordinate system.

6. A device according to any one of the preceding claims,
wherein the processing unit is configured to determine at least one of a position of the display, an orientation of the display, with reference to the X-ray coordinate system such that a position of the display with respect to the X-ray system can be determined.

7. A device according to any one of the preceding claims,
wherein the processing unit is configured for transforming the estimated actual bone position of the body part into the X-ray coordinate system and is configured to compare the estimated actual bone position with the target bone position in the X-ray coordinate system,
wherein the processing unit is configured for transforming the estimated actual bone position in the X-ray coordinate system and the target bone position in the X-ray coordinate system into the device coordinate system, such that the positions are displayable via the display from the viewing perspective of the device.

8. A device according to any one of the preceding claims,
wherein the processing unit is further configured to generate an articulated model of the imaged body part anatomy or geometric primitives indicating joint pose parameters,
wherein the device is configured to indicate the estimated actual bone position with at least one of an articulated model of the imaged body part anatomy or geometric primitives indicating joint pose parameters via the display.

9. A device according to any one of the preceding claims,
wherein the device is configured for providing a feedback to the user with respect to the target bone position and the difference between the actual bone position and the target bone position for positioning the body part of the patient.

10. A device according to any one of the claims 3 to 9,
wherein the second camera is arranged and configured to track eyes of the user operating the device for determining the viewing perspective of the user.

11. A device according to any one of the preceding claims,
wherein the first camera is positioned opposite to the display center, such that a parallax between the display and the first camera is reduced.

12. A computer program product, for assisting in positioning at least one body part of a patient for an X-ray acquisition,
wherein the computer program element, when being executed by a processor of a device for positioning at least a body part of a patient presented herein, is adapted to cause the device to
monitor the body part via a first camera of the device, wherein the body part is monitored from a viewing perspective of the device,
estimate an actual bone position of the body part based on one or more image captured by the first camera,
determine a target bone position,
calculate a difference between the estimated actual bone position and the target bone position,
display by the display at least one of the target bone position, the estimated actual bone position, and the calculated difference between the estimated actual bone position and the target bone position overlaid over at least one image of the body part,
adapt the displaying of the at least one of the target bone position, the estimated actual bone position, and the difference between the estimated actual bone position and the target bone position upon a change of the viewing perspective of the device.

13. An X-ray system comprising:
an X-ray source,
an X-ray detector for generating an X-ray image of at least a body part of a patient,
a device for positioning the body part of the patient for an X-ray acquisition according to claims 1 to 11,
wherein the device is configured for determining a position of the X-ray detector and a position of the X-ray source.

14. A method for assisting in positioning at least one body part of a patient for an X-ray acquisition, the method comprising the steps of
operating a device for positioning the at least one body part of the patient for the X-ray acquisition,
monitoring the body part to be positioned via a first camera, wherein the body part is monitored from a viewing perspective of the device,
estimate an actual bone position of the body part based on one or more image captured by the first camera,
determine a target bone position,
calculate a difference between the estimated actual bone position and the target bone position,
display by the display at least one of the target bone position, the estimated actual bone position, and the calculated difference between the estimated actual bone position and the target bone position overlaid over at least one image of the body part,
adapt the displaying of the at least one of the target bone position, the estimated actual bone position, and the difference between the estimated actual bone position and the target bone position upon a change of the viewing perspective of the device.

15. A method according to claim 14, further comprising at least one of the following steps
determining a position of an X-ray detector and a position of an X-ray source of an X-ray system used for the X-ray acquisition, wherein each of the positions is determined with reference to an X-ray coordinate system,
transforming the estimated actual bone position of the body part into the X-ray coordinate system,
comparing the estimated actual bone position with the target position in the X-ray coordinate system,
transforming the estimated actual bone position in the X-ray coordinate system and the target bone position in the X-ray coordinate system into a device coordinate system, such that the position are displayable via the display from the viewing perspective of the device.
